# EUROPEAN PATENT SPECIFICATION

(11) **EP 1 039 299 B1**
(45) Date of publication and mention of the grant of the patent: **17.11.2004**
(21) Application number: 00200677.3
(22) Date of filing: 28.02.2000
(51) Int. Cl.: G01N 33/68, G01N 33/52

(54) **A method of detecting protein and a kit using the same**
Verfahren zum Nachweis von Protein und Kit dafür
Procédé de détection de protéine et trousse utilisant ce procédé

(30) Priority: 24.03.1999 EP 99200917
(43) Date of publication of application: 27.09.2000
(73) Proprietor: JohnsonDiversey, Inc., Sturtevant, Wisconsin 53177-0902 (US); MERCK PATENT GmbH, 64293 Darmstadt (DE)
(72) Inventor: Baars, Edwin, Diversey Lever, 3606 AN Maarssen (NL); van den Brom, Guido Clemens, Diversey Lever, 3606 AN Maarssen (NL); Linxweiler, Winfried, Merck KgaA, 64293 Darmstadt (DE)
(74) Representative: Ebner von Eschenbach, Jennifer

(56) References cited:
- EP-A- 0 545 128
- EP-A- 0 738 891
- EP-A- 0 785 429
- WO-A-93/19152
- US-A- 4 013 416
- US-A- 4 239 495

## Description

### Field of the invention

The present invention relates to a method for monitoring the cleanliness of a surface treated with quaternary ammonium compounds by detecting protein present on the surface of a sample wherein the surface is treated with a disinfectant-type (or antimicrobial) quaternary ammonium compound, substances present on a portion of the thus-treated surface are transferred to a sampling means and subsequently, these substances are contacted with a reagent capable of forming or changing colour upon reaction with protein. The invention also relates to a kit for detecting protein using this method.

### Background of the invention

Soil (i.e. dirt or contamination) of mainly organic origin, and typically comprising protein, carbohydrate and/or fat, may be present on the surface of objects which come into contact with foodstuffs. This type of soil is generally associated with bacterial or microbial contamination which may present a risk to health. In order to visualize said soil it is convenient to use a reagent, such as certain dyes, which binds protein. By disclosing protein-containing soil, the location of the contamination can be indirectly visualised and can be targeted for effective cleaning. Consequently, methods for easily detecting protein have been developed in the past.

In most of the known methods for detection of protein, the surface of a sample is directly contacted by a reagent capable of forming colour upon reaction with protein. These known methods are less desirable because the colored material formed remains on the surface of the sample and can often hardly be removed with water.

As a result, the subject portion of the sample may be easily contaminated. Another problem associated with these known methods is that they generally require a long detection time.

WO-A-93/19152 discloses an aqueous cleaning composition which comprises a dye which is substantive to protein; water-miscible solvent; and surfactant, preferably alkoxylated surfactant. The dye binds to protein to form a visible coloured complex and thereby reveal soil, with the surfactant (and also to some extent the solvent) performing a cleaning function to remove soil. By visualising protein, soil can be targeted for cleaning.

In order to overcome the above-mentioned drawbacks, EP-A-738,891 (Konica) discloses a method to detect the presence of protein by transferring a substance to be detected from the surface of a sample to a water-absorbable portion of a sampling means (said portion comprising a water-insoluble synthetic polymer). This method solves the problem of contamination of the sample. Furthermore, the detection time for performing a good analysis could be expedited using this method. It is explicitly mentioned in this prior art document that the water-absorbable portion of the sampling means used in the method disclosed therein contains a synthetic polymer.
The method disclosed by EP-A-738,891 further includes the steps of contacting the transferred substances with a reagent capable of forming colour upon reaction with protein, and of measuring colour formed by this reaction so as to determine the presence of protein in the substance concerned.

When applying this method for hygiene monitoring, it has been found that it gives generally good results using any of the various protein detection methods described in EP-A-738,891. However, when this hygiene monitoring method was applied using the Coomassie protein detection dye method for monitoring the cleanliness of surfaces treated with quaternary ammonium compounds, it was found that residues of these compounds may negatively influence the detection method. Reason is that these compounds may react with the colour-forming reagent, resulting in a strong colour formation even in the absence of protein. This effect is called false positive reading caused by the quaternary ammonium compounds.

Furthermore, the prior art contains various documents which refer to colourant containing compositions, test devices and methods for determining the presence or concentration of proteins, such as albumin, in body fluids, such as urine.
For instance, EP-A-545,128 discloses a method, wherein a composition containing a colourant, a buffer and a hydrophobic polymeric compound is applied for measuring the presence of protein in urine. It is mentioned in this document that the number of false positive readings due to the presence of quaternary ammonium compounds, such as peptides, amino acids and creatinine, is reduced when using this composition.
In addition, DE-A-25 10 633 discloses a method for detecting proteins in urine, wherein a composition including an octahalogenated sulphophthalein, as colourant, is applied. This composition further contains a water-immiscible polypropylene glycol in order to overcome interference with N-containing compounds present in the urine sample tested and to reduce false positive reactions.

US-A-4 239 495 discloses an aqueous reagent system for the quantitative determination of protein in a biological fluid such as serum, cerebrospinal fluid and urine samples. The system contains Coomassie Brilliant Blue G-250 dye, a monobasic strong acid having a pKₐ of less than 3, a polybasic phosphonic acid and a water soluble, non-ionic surfactant characterised as being a block copolymer containing terminal polyethylene oxide segments separated by a polypropylene oxide segment.
It is noted that the prior art documents relating to methods for determining the presence of protein in urine samples, do not disclose anything about the negative influence caused by disinfectant-type quaternary ammonium compounds on the detection method for determining the presence of protein on a previously cleaned surface.

In view of the foregoing, it is an object of the present invention to eliminate, or at least reduce, this negative effect resulting in false positive readings and caused by residues of the disinfectant-type quaternary ammonium compounds on the results of this detection method, as disclosed by EP-A-738,891 (Konica).
In this connection, these effective disinfectant-type (or antimicrobial) quaternary ammonium compounds may be generally defined to be quaternary ammonium compounds having the formula (R₁)(R₂)(R₃)(R₄)N⁺X⁻ wherein R₁, R₂, R₃, and R₄ are independently a C₁-C₂₄ aliphatic group, a C₁-C₄ hydroxyaliphatic group, benzyl, C₁-C₂₄ alkyl benzyl, or halo benzyl, and X⁻ represents an anion capable of imparting water solubility or dispersibility to the compound such as chloride, bromide, iodide, sulphate, methylsulphate, and others.

We have surprisingly found that this object can be achieved when contacting the substances transferred onto the sampling means with a nonionic or zwitterionic surfactant as specified in claim 1.
We have also found that this detection method can be advantageously carried out when applying a sampling means having a water-absorbable portion comprising a water-insoluble polymer. This polymer can be a synthetic polymer or a non-synthetic polymer. Cellulose is preferably used as non-synthetic polymer.

### Definition of the invention

Accordingly, in one aspect the present invention provides a method for monitoring the cleanliness of a surface treated with quaternary ammonium compounds by detecting protein present on said surface, said method comprising the steps of:
- treating said surface with a disinfectant-type (or antimicrobial) quaternary ammonium compound;
- transferring substances present on a subject portion of the thus-treated surface to a sampling means which comprises a water absorbable portion comprising a water-insoluble polymer;
- contacting the substances transferred onto said sampling means with a reagent capable of forming or changing colour upon reaction with protein, wherein said reagent is a non-octahalogenated compound selected from the group consisting of phenolsulphonephthaleins and cresolsulphonephthaleins;
- visually determining color changed or formed by the reaction of said reagent with protein;
wherein the substances transferred onto the sampling means are also contacted with a zwitterionic surfactant or a nonionic surfactant selected from the group consisting of
(i) condensates of aliphatic carboxylic acids having 8 to 18 carbon atoms in an aliphatic chain and incorporating from 2 to 50 ethylene oxide and/or propylene oxide and/or butylene oxide units,
(ii) condensates of aliphatic alcohols having from 6 to 24 carbon atoms and incorporating from 2 to 50 ethylene oxide and/or propylene oxide and/or butylene oxide units,
(iii) condensates of alkyl phenols having 6 to 12 carbon atoms and incorporating from about 2 to 25 moles of ethylene oxide and/or propylene oxide and/or butylene oxide,
(iv) polyoxyethylene derivatives of sorbitan mono-, di-and tri-fatty acid esters wherein the fatty acid component has between 12 and 24 carbon atoms and the polyethylene chains contain between about 4 and 30 ethylene oxide units,
(v) polyoxyethylene-polyoxypropylene block copolymers having the formula:

   HO(CH₂CH₂O)ₐ(CH(CH₃)CH₂O)_{b}(CH₂CH₂O)_{c}H

   or

   HO(CH(CH₃)CH₂O)_{d}(CH₂CH₂O)ₑ(CH(CH₃)CH₂O)_{f}H

   wherein a, b, c, d, e, and f are integers from 1 to 350 reflecting the respective polyethylene oxide and polypropylene oxide blocks of said polymer, wherein the polyoxyethylene component of the block polymer is at least about 10% of the block polymer,
(vi) alkyl glycosides having formula:

   R⁴O(R⁵O)ₙ(Z¹)ₚ

   wherein R⁴ is a monovalent organic radical selected from a monovalent saturated aliphatic, unsaturated aliphatic or aromatic radical such as alkyl, hydroxyalkyl, alkenyl, hydroxyalkenyl, aryl, alkylaryl, hydroxyalkylaryl, arylalkyl, alkenylaryl, arylalkenyl having from 6 to 30 carbon atoms, wherein R⁵ is a divalent hydrocarbon radical containing from 2 to 4 carbon atoms such as ethylene, propylene or butylene (most preferably the unit (R⁵O)ₙ represents repeating units of ethylene oxide, propylene oxide and/or random or block combinations thereof); n is an integer from 0 to 12; Z¹ represents a moiety derived from a reducing saccharide containing 5 or 6 carbon atoms (most preferably a glucose unit); and p is a number from 0.5 to about 10.
   A zwitterionic surfactant suitable for use in the method of the present invention is a compound with a hydrophilic portion consisting of positively charged as well as negatively charged moieties and a hydrophobic uncharged aliphatic and/or aromatic component having about 6 to about 30 carbon atoms. This compound contains an equal number of positively and negatively charged moieties that give an overall uncharged or neutral compound having a pH-value in the range of about 1-9, more preferably 2-5.
   Examples of negatively charged moieties are anionic groups such as sulphate, sulphonate, phosphate, or phosphonate. Examples of zwitterionic head groups are sulfobetaines, taurine, esters of phosphoric acid with choline or aminoethanol.
   Suitable types of zwitterionic surfactant are 3-(3-Cholamidopropyl)-dimethylammonio-propanesulfonate (CHAPS) and 3-(3-Cholamidopropyl)dimethylammonio-2-hydroxy-1-propanesulfonate (CHAPSO).

In another aspect, the invention provides a kit for monitoring the cleanliness of a surface treated with quaternary ammonium compounds by detecting protein on said surface, said kit including a combination of a disinfectant-type (or antimicrobial) quaternary ammounium compound; a sampling means comprising a water-absorbable portion which comprises a water-insoluble polymer, for transferring substances present on the subject portion of the surface of a sample; a reagent capable of forming or changing colour upon reaction with protein, wherein said reagent is a non-octahalogenated compound selected from the group consisting of phenolsulphonephthaleins and cresolsulphonephthaleins; any a nonionic or zwitterionic surfactant as defined in claim 1. The polymer used in the present invention may be a synthetic polymer or a non-synthetic polymer, such as cellulose.

### Detailed description of the invention

The method according to the present invention is a method for monitoring the cleanliness of a surface treated with quaternary ammonium compounds by treating the surface with disinfectant-type quaternary ammonium compounds and detecting protein present on the surface.

When applying this method, it was found to be possible to mask (i.e. eliminate) the effect of up to 300 micrograms of any such quaternary ammonium compounds per sample.
On the other hand this type of quaternary ammonium compounds was found to give rise to a strong colour formation within 1 minute when no nonionic surfactant as defined above was present.
To avoid drying effects, best results are obtained when colour formation due to protein in the sample is monitored within 20 minutes after taking the sample concerned.

The method of the present invention is preferably carried out using the following procedure after treatment of the surface with disinfectant-type quaternary ammonium compounds. Substances which may contain protein are transferred from a portion of the surface of a sample to a sampling means. Subsequently, the sampling means is contacted with a previously prepared solution containing a nonionic or zwitterionic surfactant as defined above and the reagent capable of forming or changing colour upon reaction with protein, by adding said solution to the portion of the sampling means onto which the substances are transferred.
This solution, which is desirably an aqueous solution, is preferably added dropwise to the portion of the sampling means onto which substances are transferred.

Then the qualitative detection of protein is carried out by visually determining colour changed or formed by the reaction of said reagent with protein.

Preferably, a nonionic surfactant selected from the group consisting of condensates of aliphatic alcohols having from 6 to 24 carbon atoms and incorporating from 2 to 50 ethylene oxide units is used in the method of the invention. More preferably, the nonionic surfactant used in the invention is a condensate as mentioned above incorporating from 20 to 25 ethylene oxide units. Most preferably, this ethoxylate-type nonionic surfactant has an HLB-value in the range of 12-20.
When a solution containing the nonionic or zwitterionic surfactant and the reagent capable of forming colour is applied, the concentration of the nonionic surfactant in the aqueous solution is desirably in the range of 1-20%, more preferably 2-15%, by weight.

In the method of the invention, the sampling means comprises a water-insoluble (synthetic or non-synthetic) polymer to which protein containing substances present on the surface of a sample can be transferred. If this polymer is a non-synthetic polymer, it is may be cellulose, cellulose acetate or acetylated cellulose.

The sampling means for use in the method of the present invention can be of any shape as long as it has a portion having high water-absorbability.

Furthermore, it is preferable for convenience of sampling to attach a holding member to the water-absorbable portion comprising the non-synthetic polymer.
Examples of such desirable sampling means include the following:
- a swab-stick-shaped sampling means having the water-absorbable swab at the end of a stick;
- a tape-shaped sampling means which is composed of a water-absorbable portion located on a tape;
- a stamp-shaped sampling means in which a water-absorbable layer containing the non-synthetic polymer is attached on the stamping surface of a stamp-like handle;
- a filter paper-shaped sampling means containing the nonsynthetic polymer made in the form of a filter paper; and
- a strip-shaped sampling means comprising a substrate, such as a plastic film, and a water-absorbable layer containing the non-synthetic polymer and located on the substrate, said sampling means cut in the form of a strip and having a space for handling.

By using any suitable sampling means out of the above list, the step of transferring substances from the surface of a sample to the sampling means, may be carried out by means of wiping, pressure contacting and absorbing. For the method of wiping, a sampling means in the form of a cotton swab connected to a stick or a membrane filter may be suitably applied. For the method of pressure contacting, a tape-shaped or stamp-shaped sampling means may be suitable applied. Furthermore, for the method of absorbing, a swab-stick shaped or a filter paper-shaped sampling means may be effectively applied for absorbing the substances to be transferred.

The sampling means used in the method of the present invention preferably has a water-absorbable portion which is wettable with an aqueous medium. More preferably, in the method of the invention the water-absorbable portion is made wet using an aqueous medium before substances from the sample surface are transferred thereto.
As an aqueous medium suitable for wetting the absorbable portion, an isotonic sodium chloride solution, purified water such as distilled water or deionized water, an aqueous solution containing an anionic surface active agent, an aqueous 2-95% solution of water-miscible organic solvent such as acetone, ethanol, propyl alcohol or methylethyl ketone can be mentioned.

The substances transferred to the sampling means from the sample surface are contacted with a reagent capable of changing or forming colour upon reaction with protein for detecting the presence of protein in the transferred substances.
As the reagent for detecting protein, those necessary for detection according to the various protein detecting methods, such as biuret reaction method, Lowry method, Coomassie dying method, BCA method and ninhydrin reaction method, are usable in the method of the invention. The reagent for detecting protein is a non-octahalogenated compound selected from the group consisting of phenol sulfonephthaleins and cresol sulfonephthaleins. Suitable reagents are, for instance, bromophenol blue, bromocresol green, bromocresol purple, bromophenol red, bromothymol blue, and bromochlorophenol. Very good results were obtained when using bromocresol green.
The reaction of the transferred substances with the reagent can be performed at ambient temperature.

The protein detection kit for applying the above-mentioned method of the invention, includes a disinfectant-type (or antimicrobial) quaternary ammonium compound, a sampling means as defined above, a protein detecting reagent capable of reacting with protein and changing or producing colour as defined above, and a non-ionic or zwitterionic surfactant as defined above and suitable for eliminating the above-described negative effect of quaternary ammonium compounds on the protein detection method. Desirably, the protein detection kit of the invention also includes a wetting solution.

The invention is further illustrated by the following nonlimiting Examples, in which parts and percentages are by weight unless otherwise stated.
In the Examples the following abbreviations are used:
- Dehydol® TA20 -Nonionic surfactant, C16-C18 ethoxylated alcohol containing on average 20 EO groups;
- Lutensol® AT25 -Nonionic surfactant, C16-C18 ethoxylated alcohol containing on average 25 EO groups;
- Tween® 20 -Nonionic surfactant, polyoxyethylene sorbitan monolaurate;
- IPA -Isopropylalcohol
- ADBAC -Quaternary ammonium compound; n-alkyl-dimethylbenzylammoniumchloride, wherein n is C12, C14, or C16 (Arquad)
- BSA -Bovine serum albumin
- BCG -Bromocresolgreen
- CHAPS -(Cholamidopropyl)-dimethylammono-propanesulfonate (zwitterionic surfactant)

### Examples A,1-4

In these examples, the detection limit of various colourant containing solutions was determined with regard to the detection of ADBAC and BSA. The colourant present in these solutions was bromocresolgreen.
These solutions have following compositions:

| **Example nr.** | A | 1 | 2 | 3 | 4 |
|---|---|---|---|---|---|
| | %wt | %wt | %wt | %wt | %wt |
| Bromocresolgreen | 0.067 | 0.067 | 0.067 | 0.067 | 0.067 |
| Isopropylalcohol | 30.0 | 30.0 | 30.0 | 30.0 | 30.0 |
| Acetic acid | 3.2 | 3.2 | 3.2 | 3.2 | 3.2 |
| Citric acid | 0.2 | 0.2 | 0.2 | 0.2 | 0.2 |
| Ethyl acetate | 1.0 | 1.0 | 1.0 | 1.0 | 1.0 |
| Lutensol® AT25 | - | 10.0 | - | - | - |
| Dehydol® TA20 | - | - | 10.0 | - | - |
| Tween® 20 | - | - | - | 10.0 | - |
| CHAPS | - | - | - | - | 10.0 |
| Water | 65.5 | 55.5 | 55.5 | 55.5 | 55.5 |

Two levels of the quaternary ammonium compound ADBAC were applied onto stainless steel plates having a surface area of 10 cm². The ADBAC levels per stainless steel plate were: 100µg and 200µg.
The amount of ADBAC to be applied to the stainless steel plates was calculated assuming that, after applying an ADBAC containing solution, a residual water level of 1 mm remains on the surface of the stainless steel plate. Using this assumption it can be calculated that after drying 100 mg respectively 200 mg ADBAC per m² will be present on a surface after applying a solution containing 100 ppm respectively 200 ppm ADBAC for disinfecting that surface. Since a surface of 10 cm² is sampled in the present example, it can be derived that, when using a solution containing e.g. 200 ppm ADBAC, 200µg ADBAC is taken up when sampling the surface of the stainless steel plate.

The surface of these plates was sampled by applying a plastic strip having a cellulose pad on top. Before sampling, the pad on the strip was wetted with a solution consisting of 30% IPA and 70% water.
After sampling, one of the above colourant containing solutions was added to the cellulose pad.
Colour was judged (i) immediately after adding the colourant containing solutions (i.e. at t=0 minutes), (ii) after 2 minutes, and (iii) after 5 minutes.
The colour development was judged using the following signs: -, +/-, +, ++, where - means orange, +/- means slight green, + means green, and ++ means dark green. In this connection, an orange colour means absence of protein, whereas a green colour is an indication for the presence of protein, when no false positive reaction takes place.
The colour development results are shown in Table 1.

**TABLE 1**

| ADBAC-level | 100µg | | | 200µg | | |
|---|---|---|---|---|---|---|
| Measuring time | t=0 | t=2 | t=5 | t=0 | t=2 | t=5 |
| Example A | - | ++ | ++ | - | ++ | ++ |
| Example 1 | - | - | - | - | - | - |
| Example 2 | - | - | - | - | - | - |
| Example 3 | - | - | - | - | - | - |
| Example 4 | - | - | - | - | - | - |

It can be noticed that Example A wherein a solution was applied not containing any surfactant shows a dark green colour development which means a strong false positive reaction caused by the presence of the ADBAC on the plates sampled. On the other hand, in Examples 1-4 wherein solutions were applied containing various nonionic or zwitterionic surfactants as shown above, no green colour development was found. In other words, no false positive reaction was detectable in Examples 1-4. It follows that addition of the indicated surfactants to the colourant containing solution was found to effectively inhibit false positive reactions in the presence of the quaternary ammonium compound ADBAC and in the absence of any proteins.

In the following experiments the suitability of the described nonionic and zwitterionic surfactants for detection of various amounts of proteins (BSA) on surfaces was investigated in the absence of ADBAC. For this purpose, various levels of BSA were applied onto stainless steel plates having a surface area of 10 cm². The BSA-levels applied per stainless steel plate were: 20µg, 30 µg, and 50 µg.
Judgement of colour development was done using the same signs as applied in the above experiments (see Table 1). Also the meaning of the signs is the same: an orange colour means absence of protein, whereas a green colour is an indication for the presence of protein.
The colour development results are shown in Table 2.

**TABLE 2**

| BSA-level (µg) | 20 | | | 30 | | | 50 | | |
|---|---|---|---|---|---|---|---|---|---|
| Measuring time | t=0 | t=2 | t=5 | t=0 | t=2 | t=5 | t=0 | t=2 | t=5 |
| Example A | +/- | + | + | + | + | + | + | ++ | ++ |
| Example 1 | +/- | + | +/- | +/- | + | + | + | ++ | ++ |
| Example 2 | +/- | + | +/- | +/- | + | + | + | ++ | ++ |
| Example 3 | +/- | +/- | +/- | +/- | + | + | + | ++ | ++ |
| Example 4 | +/- | +/- | +/- | +/- | + | + | + | ++ | ++ |

It can be noticed that the detection limit when using the solution of Examples 1-4 is not significantly different from that when applying the solution of comparative Example A not containing any nonionic or zwitterionic surfactant.

### Example 5

In this example, experiments are described wherein the presence of BSA was tested in combination with the quaternary ammonium compound ADBAC.

First, a predetermined amount of BSA was added onto a stainless steel plate (having a surface area of 10 cm2) and after drying ADBAC was added to said plate.

The amount of ADBAC to be applied to the stainless steel plates tested was calculated using the same assumptions as applied in the above-described experiments.
The surface of 10 cm² stainless steel plates onto which various amounts of BSA and ADBAC were applied, was sampled using the colourant containing solutions of Examples 1-4.
Also the sampling method and the judging method for estimating the colour development were equal to those of Examples 1-4. The colour development was judged immediately after addition of the colourant containing solution, after 2 minutes, and after 5 minutes.
The results for BSA-levels of 50 µg, 100 µg and 150 µg are shown respectively in Tables 3, 4, and 5.

**TABLE 3**

| ADBAC-level (in µg) | 100 | | | 200 | | | 300 | | |
|---|---|---|---|---|---|---|---|---|---|
| measuring time | t=0 | t=2 | t=5 | t=0 | t=2 | t=5 | t=0 | t=2 | t=5 |
| solution of Example 1 | - | +/- | + | - | +/- | +/- | - | - | +/- |
| solution of Example 2 | - | +/- | + | - | +/- | +/- | - | - | +/- |
| solution of Example 3 | - | +/- | + | - | +/- | +/- | - | - | +/- |
| solution of Example 4 | - | +/- | + | - | +/- | +/- | - | - | +/- |

**TABLE 4**

| ADBAC-level (in µg) | 100 | | | 200 | | | 300 | | |
|---|---|---|---|---|---|---|---|---|---|
| measuring time | t=0 | t=2 | t=5 | t=0 | t=2 | t=5 | t=0 | t=2 | t=5 |
| solution of Example 1 | +/- | + | ++ | +/- | + | ++ | - | +/- | + |
| solution of Example 2 | +/- | + | + | - | +/- | + | - | - | +/- |
| solution of Example 3 | +/- | + | + | +/- | + | ++ | +/- | +/- | + |
| solution of Example 4 | +/- | + | + | +/- | + | ++ | +/- | +/- | + |

**TABLE 5**

| ADBAC-level (in µg) | 100 | | | 200 | | | 300 | | |
|---|---|---|---|---|---|---|---|---|---|
| measuring time | t=0 | t=2 | t=5 | t=0 | t=2 | t=5 | t=0 | t=2 | t=5 |
| solution of Example 1 | +/- | + | ++ | +/- | + | ++ | - | +/- | + |
| solution of Example 2 | +/- | + | + | - | +/- | + | - | - | +/- |
| solution of Example 3 | +/- | + | + | +/- | + | ++ | +/- | +/- | + |
| solution of Example 4 | +/- | + | + | +/- | + | ++ | +/- | +/- | + |

From the results shown in Tables 3-5, it can be derived that using the colourant containing solutions of Examples 1-4 the protein BSA can be detected in the presence of quaternary ammonium compounds after at least 2 minutes.

## Claims

1. A method for monitoring the cleanliness of a surface treated with quaternary ammonium compounds by detecting protein present on said surface, said method comprising the steps of:
- treating said surface with a disinfectant-type (or antimicrobial) quaternary ammonium compound;
- transferring substances present on a subject portion of the thus-treated surface to a sampling means which comprises a water absorbable portion comprising a water-insoluble polymer;
- contacting the substances transferred onto said sampling means with a reagent capable of forming or changing colour upon reaction with protein,wherein said reagent is a non-octdhalogenated compound selected from the group consisting of phenolsulphonephthaleins and cresolsulphonephthaleins;
- visually determining color changed or formed by the reaction of said reagent with protein;
wherein the substances transferred onto the sampling means are also contacted with a nonionic surfactant selected from the group consisting of
(i) condensates of aliphatic carboxylic acids having 8 to 18 carbon atoms in an aliphatic chain and incorporating from 2 to 50 ethylene oxide and/or propylene oxide and/or butylene oxide units,
(ii) condensates of aliphatic alcohols having from 6 to 24 carbon atoms and incorporating from 2 to 50 ethylene oxide and/or propylene oxide and/or butylene oxide units,
(iii) condensates of alkyl phenols having 6 to 12 carbon atoms and incorporating from 2 to 25 moles of ethylene oxide and/or propylene oxide and/or butylene oxide,
(iv) polyoxyethylene derivatives of sorbitan mono-, di-and tri-fatty acid esters wherein the fatty acid component has between 12 and 24 carbon atoms and the . polyethylene chains contain between 4 and 30 ethylene oxide units,
(v) polyoxyethylene-polyoxypropylene block copolymers having the formula:
HO(CH₂CH₂O)ₐ(CH(CH₃)CH₂O)_{b}(CH₂CH₂O)_{c}H
or
HO(CH(CH₃)CH₂O)_{d}(CH₂CH₂O)ₑ(CH(CH₃)CH₂O)_{f}H
wherein a, b, c, d, e, and f are integers from 1 to 350 reflecting the respective polyethylene oxide and polypropylene oxide blocks of said polymer, wherein the polyoxyethylene component of the block polymer is at least about 10% of the block polymer,
(vi) alkyl glycosides having formula:
R⁴O(R⁵O)ₙ(Z¹)ₚ
wherein R⁴ is a monovalent organic radical, selected from a monovalent saturated aliphatic, unsaturated aliphatic or aromatic radical such as alkyl, hydroxyalkyl, alkenyl, hydroxyalkenyl, aryl, alkylaryl, hydroxyalkylaryl, arylalkyl, alkenylaryl, arylalkenyl having from 6 to 30 carbon atoms, wherein R⁵ is a divalent hydrocarbon radical containing from 2 to 4 carbon atoms such as ethylene, propylene or butylene (most preferably the unit (R⁵O)ₙ represents repeating units of ethylene oxide, propylene oxide and/or random or block combinations thereof); n is an integer from 0 to 12; Z¹ represents a moiety derived from a reducing saccharide containing 5 or 6 carbon atoms (most preferably a glucose unit); and p is a number from 0.5 to 10,
or a zwitterionic surfactant, or mixtures of these surfactants.

2. Method according to claim 1, wherein the nonionic surfactant is selected from the group consisting of condensates of aliphatic alcohols including from 6 to 24 carbon atoms, incorporating from 20 to 25 moles of ethylene oxide and having an HLB-value in the range from 12-20.

3. The method according to claim 1, wherein the nonionic or zwitterionic surfactant and the reagent capable of forming colour upon reaction with protein are contained in a solution, and wherein the steps of contacting the substances transferred onto said sampling means with said reagent and with said nonionic or zwitterionic surfactant are carried out by adding said solution to the portion of the sampling means on to which the substances are transferred.

4. The method according to claim 3, wherein the solution containing the nonionic or zwitterionic surfactant and the reagent is added dropwise to the portion of the sampling means onto which the substances are transferred.

5. The method according to claim3 or 4, wherein the solution contains from 1 to 20% wt, preferably from 2 to 15% wt, of the nonionic or zwitterionic surfactant.

6. The method according to any of claims 1-5, wherein the water-absorbable portion of the sampling means is wetted with a wetting fluid in advance of the transferring step.

7. A kit for monitoring the cleanliness of a surface treated with quaternary ammonium compounds by detecting protein on said surface, said kit including a combination of a disinfectant-type (or antimicrobial) quaternary ammonium compound; a sampling means comprising a water-absorbable portion which comprises a water-insoluble polymer, for transferring substances present on the subject portion of the surface of a sample, a reagent capable of forming or changing colour upon reaction with protein; and a nonionic or zwitterionic surfactant as defined in claim 1, wherein said reagent capable of forming or changing colour upon reaction with protein is a non-octahalogenated compound selected from the group consisting of phenolsulphonephthaleins and cresolsulphonephthaleins.

8. A kit according to claim 7, wherein the reagent and the nonionic or zwitterionic surfactant are components of a solution.

## Patentansprüche

1. Verfahren zur Kontrolle der Sauberkeit einer mit quaternären Ammoniumverbindungen behandelten Oberfläche durch Nachweis von auf dieser Oberfläche vorhandenem Protein, welches Verfahren die Schritte umfasst:
- Behandeln der Oberfläche mit einer quaternären Ammoniumverbindung vom Typ eines Desinfektionsmittels (oder vom antimikrobiellen Typ);
- Übertragen von Substanzen, die auf einem betreffenden Abschnitt der so behandelten Oberfläche vorhanden sind, zu einer Probenahmevorrichtung, die einen wasserabsorbierbaren Anteil aufweist, der ein wasserunlösliches Polymer aufweist;
- Kontaktieren der auf die Probenahmevorrichtung übertragenen Substanzen mit einem Reagens, das zum Erzeugen oder zur Änderung einer Farbe bei Reaktion mit Protein in der Lage ist, worin das Reagens eine nicht octahalogenierte Verbindung ist, die ausgewählt ist aus der Gruppe, bestehend aus Phenolsulfonphthaleinen und Kresolsulfonphthaleinen;
- visuelle Bestimmung der Farbe, die durch die Reaktion des Reagens mit Protein verändert oder erzeugt wurde;
wobei die auf die Probenahmevorrichtung übertragenen Substanzen auch mit einem nichtionischen Tensid kontaktiert werden, das ausgewählt ist aus der Gruppe, bestehend aus:
(i) Kondensaten aliphatischer Carbonsäuren mit 8 bis 18 Kohlenstoffatomen in einer aliphatischen Kette, in die 2 bis 50 Ethylenoxid- und/oder Propylenoxidund/oder Butylenoxid-Einheiten eingebaut sind;
(ii) Kondensaten aliphatischer Alkohole mit 6 bis 24 Kohlenstoffatomen, in die 2 bis 50 Ethylenoxid- und/oder Propylenoxid- und/oder Butylenoxid-Einheiten eingebaut sind;
(iii) Kondensaten von Alkylphenolen mit 6 bis 12 Kohlenstoffatomen, in die 2 bis 25 Mol Ethylenoxid und/oder Propylenoxid und/oder Butylenoxid eingebaut sind;
(iv) Polyoxyethylen-Derivaten von Sorbitanmono-, -di- und -tri-Fettsäureestern, worin die Fettsäure-Komponente 12 bis 24 Kohlenstoffatome hat und die Polyethylenketten zwischen 4 und 30 Ethylenoxid-Einheiten enthalten;
(v) Polyoxyethylen/Polyoxypropylen-Blockcopolymeren mit der Formel:
HO(CH₂CH₂O)ₐ(CH(CH₃)CH₂O)_{b}(CH₂CH₂O)_{c}H
oder
HO(CH(CH₃)CH₂O)_{d}(CH₂CH₂O)ₑ(CH(CH₃)CH₂O)_{f}H
worin a, b, c, d, e und f ganze Zahlen von 1 bis 350 sind die die entsprechenden Polyethylenoxid- und Polypropylenoxid-Blöcke des Polymers wiedergeben, worin die Polyoxyethylen-Komponente des Blockpolymers mindestens etwa 10% des Blockpolymers ausmacht;
(vi) Alkylglykosiden der Formel:
R⁴O(R⁵O)ₙ(Z¹)ₚ
worin R⁴ ein einwertiger organischer Rest ist, ausgewählt aus einem einwertigen, gesättigten aliphatischen, ungesättigten aliphatischen oder aromatischen Rest, wie beispielsweise Alkyl, Hydroxyalkyl, Alkenyl, Hydroxyalkenyl, Aryl, Alkylaryl, Hydroxyalkylaryl, Arylalkyl, Alkenylaryl, Arylalkenyl, die 6 bis 30 Kohlenstoffatome haben, worin R⁵ ein zweiwertiger Kohlenwasserstoff-Rest ist, der 2 bis 4 Kohlenstoffatome enthält, wie beispielsweise Ethylen, Propylen oder Butylen (am meisten bevorzugt die Einheit (R⁵O)ₙ, die repetierende Einheiten von Ethylenoxid, Propylenoxid und/oder Random- oder Blockkombinationen davon repräsentiert); n ist eine ganze Zahl von Null bis 12; Z' stellt einen Teil dar, der von einem reduzierenden Saccharid deriviert ist, der 5 bis 6 Kohlenstoffatome enthält (am meisten bevorzugt eine Glucose-Einheit); und p ist eine Zahl von 0,5 bis 10;
oder ein Amphotensid oder Mischungen dieser Tenside.

2. Verfahren nach Anspruch 1, bei welchem das nichtionische Tensid ausgewählt ist aus der Gruppe, bestehend aus Kondensaten von aliphatischen Alkoholen, die 6 bis 24 Kohlenstoffatome enthalten und in die 20 bis 25 Mol Ethylenoxid eingebaut sind und einen HLB-Wert im Bereich von 12 bis 20 haben.

3. Verfahren nach Anspruch 1, bei welchem das nichtionische Tensid oder Amphotensid und das Reagens, das zur Erzeugung einer Farbe bei Reaktion mit Protein in der Lage ist, in einer Lösung enthalten sind und bei welchem die Schritte des Kontaktierens der auf die Probenahmevorrichtung übertragenen Substanzen mit dem Reagens und mit dem nichtionischen Tensid oder Amphotensid ausgeführt werden, indem die Lösung zu dem Teil der Probenahmevorrichtung gegeben wird, auf den die Substanzen übertragen wurden.

4. Verfahren nach Anspruch 3, bei welchem die Lösung, die das nichtionische Tensid oder Amphotensid und das Reagens enthält, tropfenweise zu dem Teil der Probenahmevorrichtung gegeben wird, auf dem die Substanzen übertragen wurden.

5. Verfahren nach Anspruch 3 oder 4, bei welchem die Lösung 1 % bis 20 Gew.% und bevorzugt 2% bis 15 Gew.% des nichtionischen Tensids oder Amphotensids enthält.

6. Verfahren nach einem der Ansprüche 1 bis 5, bei welchem der wasserabsorbierbare Teil der Probenahmevorrichtung vor dem Schritt des Übertragens mit einer benetzenden Flüssigkeit benetzt wird.

7. Kit zur Kontrolle der Sauberkeit einer mit quaternären Ammoniumverbindungen behandelten Oberfläche durch Detektieren von Protein auf der Oberfläche, wobei das Kit eine Kombination einer quaternären Ammoniumverbindung vom Desinfektionstyp (oder antimikrobiellen Typ) einschließt; eine Probenahmevorrichtung, die einen wasserabsorbierbaren Teil aufweist, der ein wasserunlösliches Polymer aufweist, und zwar zur Übertragung der auf dem betreffenden Abschnitt der Oberfläche einer Probe vorhandenen Substanzen; ein Reagens, das zur Erzeugung oder zur Veränderung der Färbung bei Reaktion mit Protein in der Lage ist; und ein nichtionisches Tensid oder Amphotensid nach Anspruch 1; wobei das Reagens, das zur Erzeugung oder Änderung der Farbe bei Reaktion mit Protein in der Lage ist, eine nicht octahalogenierte Verbindung ist, die ausgewählt ist aus der Gruppe, bestehend aus Phenolsulfonphthaleinen und Kresolsulfonphthaleinen.

8. Kit nach Anspruch 7, worin das Reagens und das nichtionische Tensid oder Amphotensid Komponenten einer Lösung sind.

## Revendications

1. Procédé de contrôle de la propreté d'une surface traitée avec des composés d'ammonium quaternaires en détectant les protéines présentes sur ladite surface, ledit procédé comprenant les étapes suivantes :
- le traitement de ladite surface avec un composé d'ammonium quaternaire de type désinfectant (ou antimicrobien) ;
- le transfert des substances présentes sur une partie proposée de la surface ainsi traitée dans un dispositif d'échantillonnage qui comporte une partie absorbable dans l'eau comprenant un polymère insoluble dans l'eau ;
- la mise en contact des substances transférées sur ledit dispositif d'échantillonnage avec un réactif pouvant former une couleur ou la modifier par réaction avec la protéine, ledit réactif étant un composé non octa-halogéné choisi dans le groupe constitué de phénolsulfonephtaléines et de crésolsulfonephtaléines ;
- la détermination visuelle du changement ou de la formation de couleur par la réaction dudit réactif avec la protéine,
dans lequel les substances transférées sur le dispositif d'échantillonnage sont également mises en contact avec un tensioactif non ionique choisi dans le groupe constitué par
(i) les produits de condensation d'acides carboxyliques aliphatiques présentant 8 à 18 atomes de carbone dans une chaîne aliphatique et incluant de 2 à 50 unités d'oxyde d'éthylène et/ou d'oxyde de propylène et/ou d'oxyde de butylène,
(ii) les produits de condensation d'alcools aliphatiques présentant 6 à 24 atomes de carbone et incluant de 2 à 50 unités d'oxyde d'éthylène et/ou d'oxyde de propylène et/ou d'oxyde de butylène,
(iii) les produits de condensation de phénols d'alkyle présentant 6 à 12 atomes de carbone et incluant de 2 à 25 moles d'oxyde d'éthylène et/ou d'oxyde de propylène et/ou d'oxyde de butylène,
(iv) les dérivés polyoxyéthylène de mono-, di- et tri- esters d'acides gras sorbitan dans lesquels le composant d'acide gras comprend entre 12 et 24 atomes de carbone et les chaînes de polyéthylène contiennent entre 4 et 30 unités d'oxyde d'éthylène,
(v) les copolymères en bloc de polyoxyéthylène-polyoxypropylène présentant la formule :
HO(CH₂CH₂O)ₐ(CH (CH₃)CH₂O)_{b}(CH₂CH₂O)_{c}H
ou
HO(CH(CH₃)CH₂O)_{d}(CH₂CH₂O)ₑ(CH(CH₃)CH₂O)_{f}H
a, b, c, d, e et f étant des nombres entiers de 1 à 350 correspondant aux blocs respectifs d'oxyde de polyéthylène et d'oxyde de polypropylène dudit polymère, le composant polyoxyéthylène du polymère en bloc constituant au moins 10 % du polymère en bloc,
(vi) les glycosides d'alkyle présentant la formule :
R⁴O(R⁵O)ₙ(Z¹)ₚ
R⁴ étant un radical organique monovalent, choisi parmi un radical aliphatique monovalent saturé, un radical aliphatique insaturé ou un radical aromatique, tel que l'alkyle, l'hydroxyalkyle, l'alkényle, l'hydroxyalkényle, l'aryle, l'alkylaryle, l'hydroxyalkylaryle, l'arylalkyle, l'alkénylaryle, l'arylalkényle présentant de 6 à 30 atomes de carbone, R⁵ étant un radical d'hydrocarbure divalent contenant de 2 à 4 atomes de carbone, tel que l'éthylène, le propylène ou le butylène (de manière davantage préférée, l'unité (R⁵O)ₙ représente des unités répétitives d'oxyde d'éthylène, d'oxyde de propylène et/ou leurs combinaisons aléatoires ou en bloc) ; n est un nombre entier de 0 à 12 ; Z¹ représente une fraction dérivée d'un saccharide réducteur contenant 5 ou 6 atomes de carbone (de manière davantage préférée, une unité de glucose) ; et p est un nombre entier de 0,5 à 10,
ou un tensioactif zwitterionique, ou des mélanges de ces tensioactifs.

2. Procédé selon la revendication 1, dans lequel le tensioactif non ionique est choisi dans le groupe constitué de condensés d'alcools aliphatiques comprenant de 6 à 24 atomes de carbone, incluant de 20 à 25 moles d'oxyde d'éthylène et présentant une valeur HLB de l'ordre de 12 à 20.

3. Procédé selon la revendication 1, dans lequel le tensioactif non ionique ou zwitterionique et le réactif pouvant former une couleur par réaction avec la protéine sont contenus dans une solution, et dans lequel les étapes de mise en contact des substances transférées sur ledit dispositif d'échantillonnage avec ledit réactif et avec ledit tensioactif non ionique ou zwitterionique sont réalisées en ajoutant ladite solution à la partie du dispositif d'échantillonnage sur lequel les substances sont transférées.

4. Procédé selon la revendication 3, dans lequel la solution contenant le tensioactif non ionique ou zwitterionique et le réactif, est ajoutée goutte à goutte à la partie du dispositif d'échantillonnage sur lequel les substances sont transférées.

5. Procédé selon la revendication 3 ou 4, dans lequel la solution contient de 1 à 20 % en poids, de préférence de 2 à 15 % en poids de tensioactif non ionique ou zwitterionique.

6. Procédé selon l'une quelconque des revendications 1 à 5, dans lequel la partie absorbable dans l'eau du dispositif d'échantillonnage est mouillée avec un fluide mouillant avant l'étape de transfert.

7. Kit de contrôle de la propreté d'une surface traitée avec des composés d'ammonium quaternaires en détectant les protéines présentes sur ladite surface, ledit kit comprenant une combinaison d'un composé d'ammonium quaternaire de type désinfectant (ou antimicrobien); un dispositif d'échantillonnage comprenant une partie absorbable dans l'eau qui comprend un polymère insoluble dans l'eau, pour transférer des substances présentes sur la présente partie de la surface d'un échantillon ; un réactif pouvant former une couleur ou la modifier par réaction avec la protéine, et un tensioactif non ionique ou zwitterionique tel qu'il est défini à la revendication 1, dans lequel ledit réactif pouvant former ou modifier la couleur par réaction avec la protéine est un composé non octa-halogéné choisi dans le groupe constitué de phénolsulfonephtaléines et de crésolsulfonephtaléines.

8. Kit selon la revendication 7, dans lequel le réactif et le tensioactif non ionique ou zwitterionique sont des composants de la solution.
